# EUROPEAN PATENT SPECIFICATION

(11) **EP 4 154 917 B1**
(45) Date of publication and mention of the grant of the patent: **14.08.2024**
(21) Application number: 22197826.5
(22) Date of filing: 26.09.2022
(51) Int. Cl.: A61L 2/10, E03D 9/00

(54) **SYSTEMS AND METHODS FOR ULTRAVIOLET LIGHT DISINFECTING TREATMENTS**
SYSTEME UND VERFAHREN FÜR DESINFEKTIONSBEHANDLUNGEN MIT ULTRAVIOLETTEM LICHT
SYSTÈMES ET PROCÉDÉS DE TRAITEMENT DE DÉSINFECTION PAR LUMIÈRE ULTRAVIOLETTE

(30) Priority: 24.09.2021 US 202117485053
(43) Date of publication of application: 29.03.2023
(73) Proprietor: B/E Aerospace, Inc., Winston-Salem, NC 27105 (US)
(72) Inventor: EDQUIST, John, Milwaukee, WI (US)
(74) Representative: Dehns

(56) References cited:
- WO-A1-2021/178782
- US-B2- 10 967 081

## Description

### FIELD

The present disclosure relates to disinfecting systems and methods, and, in particular, to systems and methods for disinfecting a lavatory using ultraviolet (UV) lighting.

### BACKGROUND

The recent novel-coronavirus (SARS-COV-2) outbreak has negatively impacted the safety and health of many people. Pathogens can be transmitted via direct airborne transmission between users or via indirect contact transmission from different users occupying the same space at different times. For example, lingering pathogens may remain on contact surfaces of an aircraft lavatory to be spread to subsequent lavatory occupants. The presence of pathogens on aircraft lavatory surfaces may be reduced by performing ultraviolet (UV) lighting disinfecting treatments to the lavatory surfaces. However, the UV lighting disinfecting system generally does not operate while the lavatory is occupied. Accordingly, if a series of passengers enter the lavatory one after another, the length of time to reach a target dosage on the lavatory surfaces may not be achieved. US 10,967,081 B2 describes UV germicidal devices, systems and methods. WO 2021/178782 A1 describes a UV disinfection platform.

### SUMMARY

A disinfecting system for a lavatory is disclosed herein. In accordance with various embodiments, the disinfecting system may comprise an ultraviolet light source, a controller in operable communication with the ultraviolet light source, and a lavatory sensor operably coupled to the controller. The controller may be configured to track a number of consecutive incomplete dosage cycles. The controller may implement a first dosage cycle if the number of consecutive incomplete dosage cycles is less than a threshold number. The controller may implement a second dosage cycle if the number of consecutive incomplete dosage cycles is greater than or equal to the threshold number.

In various embodiments, the lavatory sensor comprises at least one of a flush sensor, a door latch position sensor, a motion sensor, or a light sensor. In various embodiments, the first dosage cycle is configured to achieve a 1 log reduction of a target pathogen. In various embodiments, the second dosage cycle is configured to achieve a 3 log reduction of the target pathogen.

In various embodiments, the controller is configured to command the ultraviolet light source to turn off prior to completion of at least one of the first dosage cycle or the second dosage cycle in response receiving a signal from the lavatory sensor indicating an occupant has entered the lavatory.

In various embodiments, the controller is configured to add one to the number of consecutive incomplete dosage cycles if the ultraviolet light source is commanded by the controller to turn off prior to completion of at least one of the first dosage cycle or the second dosage cycle.

In various embodiments, the controller is configured to set the number of consecutive incomplete dosage cycles at zero if at least one of the first dosage cycle or the second dosage cycle is completed.

An article of manufacture including a tangible, non-transitory computer-readable storage medium having instructions stored thereon for controlling a disinfecting system for a lavatory is also disclosed herein. In accordance with various embodiments, the instructions, in response to execution by a controller, cause the controller to perform operations, which may comprise identifying, by the controller, a disinfection initiation event; comparing, by the controller, a consecutive skipped cycles count to a threshold number; and commanding, by the controller, a ultraviolet light source to implement at least one of a first dosage cycle or a second dosage cycle based on the comparison of the consecutive skipped cycles count to the threshold number. The controller may be configured to implement the first dosage cycle if the skipped cycle count is less than the threshold number. The controller may be configured to implement the second dosage cycle if the consecutive skipped cycles count is greater than or equal to the threshold number.

In various embodiments, the operations may further comprise starting, by the controller, a motion timer, in response to identifying the disinfection initiation event. In various embodiments, the operations may further comprise adding, by the controller, one to the consecutive skipped cycles count if an interruption event is detected by the controller prior to the motion timer exceeding a threshold time.

In various embodiments, the operations may further comprise receiving, by the controller, a signal from a lavatory sensor configured to detect at least one of a toilet flush, a door latch position, motion in the lavatory, or a level of light in the lavatory.

In various embodiments, the controller may be configured to identify the disinfection initiation event based on the signal from the lavatory sensor.

In various embodiments, the operations may further comprise identifying, by the controller, an interruption event based on the signal from the lavatory sensor; commanding, by the controller, the ultraviolet light source to stop the at least one of the first dosage cycle of the second dosage cycle in response to identifying the interruption event; and adding, by the controller, one to the consecutive skipped cycles count.

In various embodiments, the operations may further comprise setting, by the controller, the consecutive skipped cycles count to zero if at least one of the first dosage cycle or the second dosage cycle is completed.

In various embodiments, the operations may further comprise commanding, by the controller, a display to output a message conveying an amount of time before the at least one of the first dosage cycle or the second dosage cycle is completed.

In various embodiments, the first dosage cycle may be configured to achieve a 1 log reduction of a target pathogen, and the second dosage cycle may be configured to achieve a 3 log reduction of the target pathogen.

A method disinfecting a lavatory is also disclosed herein. In accordance with various embodiments, the method may comprise the step of identifying a disinfection initiation event, starting a motion timer, comparing a consecutive skipped cycles count to a threshold number if the motion timer exceeds a threshold time, and implementing at least one of a first dosage cycle or a second dosage cycle based on the comparison of the consecutive skipped cycles count to the threshold number. The first dosage cycle is implemented if the consecutive skipped cycles count is less than the threshold number, and the second dosage cycle is implemented if the consecutive skipped cycles count is greater than or equal to the threshold number.

In various embodiments, the method may further comprise adding one to the consecutive skipped cycles count if an interruption event is detected prior to the motion timer exceeding the threshold time.

In various embodiments, the method may further comprise adding one to the consecutive skipped cycles count if an interruption event is detected prior to the at least one of the first dosage cycle or the second dosage cycle being completed.

In various embodiments, the method may further comprise setting the consecutive skipped cycles count to zero if the at least one of the first dosage cycle or the second dosage cycle is completed.

The foregoing features and elements may be combined in various combinations without exclusivity, unless expressly indicated otherwise. These features and elements as well as the operation thereof will become more apparent in light of the following description and the accompanying drawings. It should be understood, however, the following description and drawings are intended to be exemplary in nature and non-limiting.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the detailed description and claims when considered in connection with the figures, wherein like numerals denote like elements.
FIG. 1 illustrates an aircraft having a lavatory, in accordance with various embodiments;
FIG. 2 illustrates an aircraft lavatory having a UV light disinfecting system, in accordance with various embodiments;
FIG. 3 illustrates a schematic of a UV light disinfecting system for sanitization of an aircraft lavatory, in accordance with various embodiments; and
FIG. 4 illustrates a process for sanitizing an aircraft lavatory using a UV light disinfecting system, in accordance with various embodiments.

### DETAILED DESCRIPTION

The detailed description of exemplary embodiments herein makes reference to the accompanying drawings, which show exemplary embodiments by way of illustration.

Furthermore, any reference to singular includes plural embodiments, and any reference to more than one component or step may include a singular embodiment or step. Also, any reference to attached, fixed, connected or the like may include permanent, removable, temporary, partial, full and/or any other possible attachment option. Surface shading lines may be used throughout the figures to denote different parts but not necessarily to denote the same or different materials.

The present disclosure is directed to systems and methods for sanitization of an aircraft lavatory using a UV light disinfecting system. The systems and methods herein include one or more UV light source(s) targeted toward the surfaces of an aircraft lavatory. The duration of time the UV light source is powered on (i.e., the disinfecting dosage) is determined by a controller and can be implemented dynamically. In accordance with various embodiments, the controller tracks lavatory usage cycles based on signals received from one or more lavatory sensors. For example, the controller may monitor toilet flush, a door latch, an ambient light level, movement within the lavatory, etc. If a disinfecting cycle is repeatedly interrupted before completing a desired disinfecting dosage, the controller will implement a greater dosage cycle at the next opportunity to achieve a greater percent reduction of pathogens. In accordance with various embodiments, the disinfecting dose (e.g., duration of the time the UV light source powered on) for a normal cycle and the disinfecting dose for the extending cycle are configurable based on operator specifications such as a desired percent of disinfection (e.g., 1 log, 2 log, 3 log, etc.), a target pathogen to be injured, neutralized, killed, or the like.

In accordance with various embodiments, UV light sources of the present disclosure may emit UV-C light. This light may be defined as a germicidal light source having a peak wavelength that is between 200 nanometers (nm) and 280 nm, between 210 nm and 225 nm, or about 222 nm. Where used in this context, "about" refers to the referenced value plus or minus 10 percent of the referenced value. UV light of this type may effectively injure, neutralize, or kill pathogens that are both airborne and resting on surfaces. In addition, this light may be readily absorbed by most materials and may be relatively safe for human exposure.

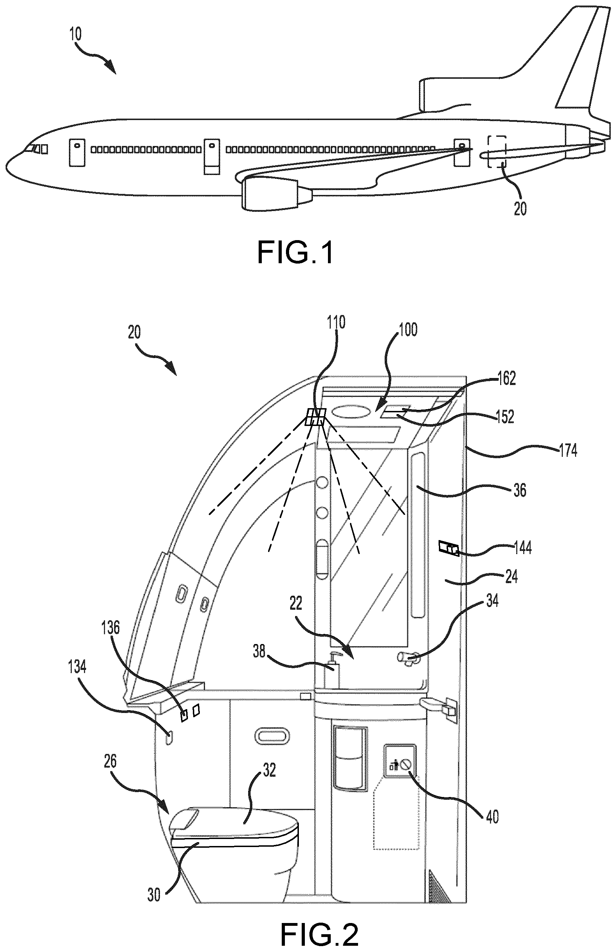

In various embodiments, and with reference to FIGs. 1 and 2, an aircraft 10 may include aircraft lavatory 20. The aircraft lavatory 20 may include a washbasin (e.g., a sink) 22, a door 24, and a toilet 26, among other features. A disinfecting system 100 may be installed in lavatory 20. Disinfecting system 100 includes a UV light source 110 configured to emit a UV-C light. In this regard, UV light source 110 may comprise an excimer lamp, a light emitting diode (LED), or the like configured to emit UV-C light. In various embodiments, the UV-C light may be directional light (i.e., oriented and directed in a specific direction) or it may be non-directional light. The present disclosure is not limited in this regard. Disinfecting system 100 is configured to disinfect, or sanitize, lavatory 20 during in-flight cycle, post-flight cycle, or the like. As described in further detail below, disinfecting system 100 is configured to disinfect lavatory 20 after use (e.g., in response to detecting a user entering lavatory 20 and detecting the user leaving lavatory 20).

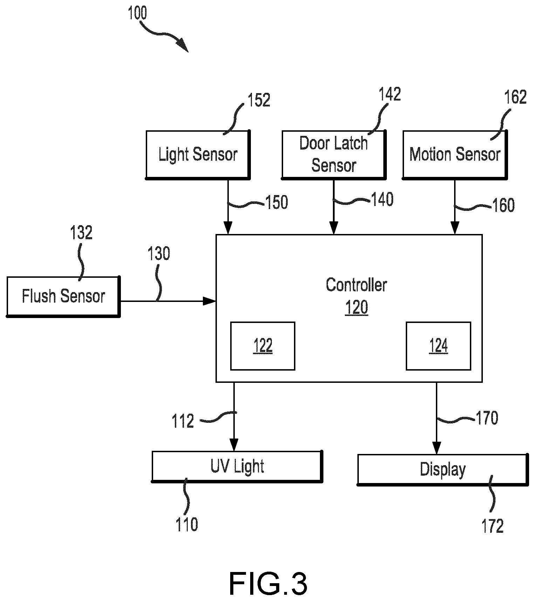

FIG. 3 illustrates a schematic of disinfecting system 100. With combined reference to FIG. 2 and FIG. 3, in various embodiments, UV light source 110 is operably coupled to a lavatory component controller 120. Controller 120 is configured command UV light source 110 to implement one or more dosage cycles. In this regard, controller 120 is configured to command and/or cause UV light source 110 to turn on and to turn off. Controller 120 may include a general-purpose processor, a digital signal processor (DSP), an application specific integrated circuit (ASIC), a field programmable gate array (FPGA), or some other programmable logic device, discrete gate or transistor logic, discrete hardware components, or any combination thereof. A tangible, non-transitory computer-readable storage medium 122 may be in communication with controller 120. The storage medium 122 may comprise any tangible, non-transitory computer-readable storage medium known in the art. The storage medium 122 has instructions stored thereon that, in response to execution by controller 120, cause controller 120 to perform operations related to controlling disinfecting system 100. Controller 120 may also control the function of one or more other lavatory components. For example, controller 120 may control one more of a flushing of toilet 26, actuation (e.g., a raising and a lowering) of a toilet seat 30 and/or of a toilet lid 32 of toilet 26, the flow of water to a faucet 34 of sink 22, turning lavatory lights 36 on and off, dispensing of soap from a soap dispenser 38, actuation of a waste receptacle cover 40, and/or other "touchless" features of lavatory 20.

Controller 120 is configured to receive signals from one or more lavatory sensors. For example, controller 120 may receive a flush signal 130 from a toilet sensor 132. Flush signal 130 may be sent in response to a flush actuator 134 being manually actuated by an occupant of lavatory 20. Flush signal 130 may also be sent in response to toilet being electrically flushed by controller 120. For example, a toilet position sensor 136 may detect a proximity of an occupant to toilet seat 30. Controller 120 may determine an occupant has moved away from toilet 26 based on the signals from toilet position sensor 136. Controller 120 may command toilet 26 to flush in response to determining the occupant has moved away from toilet 26. In either case, flush signal 130 is sent to controller 120 in response to a flushing of toilet 26.

In various embodiments, controller 120 may be configured to receive a door latch position signal 140 from a door latch sensor 142. Door latch sensor 142 is configured to detect a position of a door latch 144 on door 24. In various embodiments, lavatory lights 36 may turn on and turn off in response to actuation of door latch 144. For example, lavatory lights 36 may be powered on in response to door latch 144 being translated to an "occupied" or "locked" position. Lavatory lights 36 may be powered off in response to door latch 144 being translated to an "unoccupied" or "unlocked" position.

In various embodiments, controller 120 may be configured to receive a light detection signal 150 from a light sensor 152. Light sensor 152 is configured to detect ambient light in lavatory 20 and/or light output by lavatory lights 36. Light sensor 152 may be configured to detect visible, or non-ultraviolet, light. For example, light sensor 152 may be configured to detect light having a wavelength approximately equal to the light output by lavatory lights 36. In various embodiments, light sensor 152 may detect light having a wavelength between 400 nm and 700 nm.

In various embodiments, controller 120 may receive a motion detected signal 160 from motion sensor 162. Motion sensor 162 is configured to detect motion within lavatory 20. Motion sensor 162 may include a passive infrared (PIR) sensor, an active or passive ultrasonic sensor, a microwave sensor, tomographic sensor, and/or any other sensor capable of detecting motion within lavatory 20 and/or combinations thereof.

In accordance with various embodiments, controller 120 is configured to send commands 112 to UV light source 110. Controller 120 is configured to command UV light source 110 to turn on, in response to determining an occupant has exited lavatory 20. Controller 120 is further configured to command UV light source 110 to turn off in response to determining an occupant has entered lavatory 20. Turning UV light source 110 off prior to completion of a disinfection cycle can reduce the dosage (e.g., pathogen killing ability) of UV light source 110. However, completing multiple shorter cycles within a defined period may achieve the desired pathogen reduction. For example, running three (3) separate thirty second disinfection cycle with each thirty second disinfection cycle generating a 1.0 millijoule per square centimeter (mJ/cm²) dose may achieve a 3 log (99.9%) reduction in SARS-COV-2 pathogens. Similarly, running one ninety second cycle generating a 3.0 mJ/cm² dose may achieve a 3 log (99.9%) reduction in SARS-COV-2 pathogens. It is contemplated and understood that the previous doses are exemplary for SARS-COV-2, and that disinfecting system 100 may be configured to generate other doses based on the target pathogen.

In accordance with various embodiments, controller 120 is configured to run a disinfection cycle each time controller 120 determines an occupant has exited lavatory 20. Controller 120 may then monitor if the disinfection cycle is completed. Controller 120 may track the number of consecutive incomplete disinfection cycles, and if the number of consecutive incomplete disinfection cycles exceeds a threshold number, controller 120 may run an extended cycle. In this regard, controller 120 may be configured to choose between a normal (or first) length cycle and an extended (or second) length cycle based on the number of consecutive incomplete disinfection cycles. In various embodiments, the normal cycle is configured to achieve a 1 log (i.e. 90%) reduction of the target pathogen, and the extended cycle is configured to achieve a 3 log (i.e., 99.9%) reduction in the target pathogen. In various embodiments, the power (e.g., voltage) provided by controller 120 to UV light source 110 during the normal cycle may be approximately equal to the power provided to UV light source 110 during the extended cycle, with the power being provided to UV light source 110 for a longer duration of time in the extended cycle. In other words, the brightness or intensity of light source 110 may be the same in the normal cycle and the extended cycle, with the increased energy/area generated during the extended cycle being produced by having the light source 110 powered on for a longer duration of time as compared to the normal cycle.

In various embodiments, controller 120 may send display commands 170 to a display 172. Display 172 may be located outside lavatory 20. Display 172 may be visible to a person trying to enter lavatory 20. For example, display 172 may be located on an outside surface 174 of door 24. Outside surface 174 is oriented away from lavatory 20, when door 24 is closed. In various embodiments, display may be on a wall, panel, or door frame proximate door 24. In various embodiments, display 172 is configured to convey if a disinfection cycle is taking place. In various embodiments, display 172 may comprise a countdown feature configured to convey how many seconds are remaining in the current disinfection cycle. Thus, display 172 may allow a passenger to make a more informed decision regarding whether he/she wants to wait for the disinfection cycle to finish or to enter the lavatory immediately.

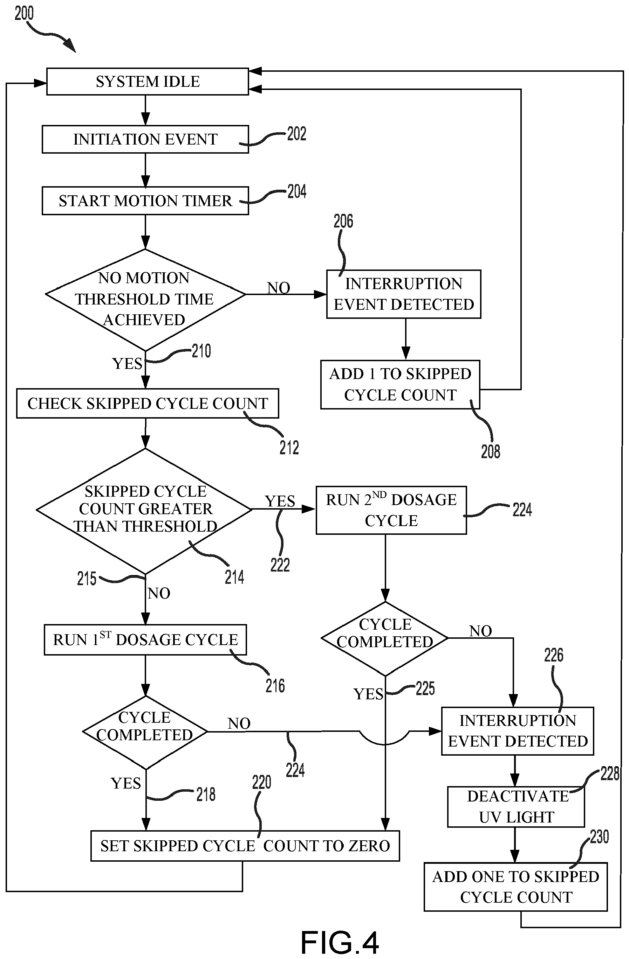

With reference to FIG. 4, a process 200 for sanitizing an aircraft lavatory using a UV light disinfecting system is illustrated. Process 200 may be performed by controller 120, with momentary reference to FIG. 3. Controller 120 may employ components of disinfecting system 100 and/or lavatory 20, with momentary reference to FIGs. 2 and 3 to perform process 200.

With combined reference to FIG. 4 and FIG. 3, process 200 may begin in response to controller 120 identifying a cycle initiation event (step 202). For example, step 202 may include controller 120 receiving at least one of a flush signal 130, a door latch position signal 140 indicating door latch 144 has translated from the locked position to the unlocked position, or a light detection signal 150 indicating lavatory lights 36 are turned off. In response to the identifying the cycle initiation event, controller 120 may start a motion timer 124 (step 204). Motion timer 124 may be configured to monitor a duration of time in which no motion is detected in lavatory 20. In this regard, motion timer 124 may stop counting (counting up or counting down) in response to controller 120 receiving a motion detected signal 160 from motion sensor 162.

If controller 120 identifies an interruption invent (step 206) before the motion timer has reached the threshold time, controller 120 adds one (1) to the skipped cycle count (step 208). The skipped cycle count may be stored in storage medium 122. Step 206 may include controller 120 receiving a signal from a lavatory sensor indicating an occupant has entered lavatory 20. For example, step 206 may include controller 120 receiving at least one of a motion detected signal 160 from motion sensor 162, a door latch position signal 140 indicating door latch 144 has translated from the unlocked position to the locked position, a light detection signal 150 indicating lavatory lights 36 are turned on.

If controller 120 determines the motion timer has reached a threshold time (determination 210), controller 120 checks the skipped cycle count (step 212). Step 212 may include controller 120 comparing the skipped cycle count to a threshold number (determination 214). If the skipped cycle count is less than the threshold number (determination 215), for example, if the skipped cycle count is less than 3, less than 5, less than 10, or any other number which may be set by the aircraft operator, controller 120 commands UV light source 110 to turn on for the normal (or first) dosage cycle (step 216). In various embodiments, the normal dosage cycle is configured to achieve a 1 log reduction in a target pathogen. If the normal dosage cycle is completed without interruption (determination 218), controller 120 sets the skipped cycle count to zero (step 220).

If controller 120 determines that the skipped cycle count is greater than or equal to the threshold number (determination 222), controller 120 commands UV light source 110 to turn on for the extended (or second) dosage cycle (step 224). In various embodiments, the extended dosage cycle is configured to achieve a 3 log reduction in a target pathogen. If the extended dosage cycle is completed without interruption (determination 225), controller 120 sets the skipped cycle count to zero (step 220).

If controller 120 identifies an interruption invent (step 226) before either the normal dosage cycle or the extended dosage cycle is completed, controller 120 commands UV light source 110 to turn off (step 228) and adds one (1) the skipped cycle count (step 230). Step 228 may include controller 120 receiving a signal from a lavatory sensor indicating an occupant has entered lavatory 20. For example, step 227 may include controller 120 receiving at least one of a motion detected signal 160 from motion sensor 162, a door latch position signal 140 indicating door latch 144 has translated from the unlocked position to the locked position, a light detection signal 150 indicating lavatory lights 36 are turned on.

In various embodiments, in response to initiating the normal dosage cycle (step 216) or the extended dosage cycle (step 224), controller 120 may command to display 172 (FIG. 3) output a message configured to convey that a disinfection cycle is taking place. In various embodiments, the message output by display 172 may include a countdown clock configured to convey to passengers how much time is remains before the disinfection cycle is complete.

Disinfecting system 100 and/or process 200 allows controller 120 to implement a disinfection dosage dynamically, based on sensor input correlation to the number of consecutively missed cycles (e.g., the number of occupants between sanitization). This enables the lavatory to receive the same desired UV dose after several users to reduce likelihood of pathogen buildup from multiple users. It also reduces a need for intervention cleaning from crew members. The message on display 172 allows passengers to make an informed choice with regard to entering the lavatory immediately after the previous person or choosing to wait for a disinfection cycle to be completed.

Benefits and other advantages have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, and any elements that may cause any benefit or advantage to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C.

Systems, methods, and apparatus are provided herein. In the detailed description herein, references to "various embodiments", "one embodiment", "an embodiment", "an example embodiment", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Furthermore, no element, component, or method step in the present disclosure is intended to be dedicated to the public regardless of whether the element, component, or method step is explicitly recited in the claims. As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

## Claims

1. A disinfecting system for a lavatory, comprising:
an ultraviolet light source (110);
a controller (120) in operable communication with the ultraviolet light source (110); and
a lavatory sensor operably coupled to the controller (120), wherein the controller (120) is configured to track a number of consecutive skipped cycles, and wherein the controller (120) is configured to implement a first dosage cycle if the number of consecutive skipped cycles is less than a threshold number, and wherein the controller (120) is configured to implement a second dosage cycle if the number of consecutive skipped cycles is greater than or equal to the threshold number.

2. The disinfecting system of claim 1, wherein the lavatory sensor comprises at least one of a flush sensor, a door latch position sensor, a motion sensor, or a light sensor.

3. The disinfecting system of claim 2, wherein the first dosage cycle is configured to achieve a 1 log reduction of a target pathogen, and/or wherein the second dosage cycle is configured to achieve a 3 log reduction of the target pathogen.

4. The disinfecting system of any preceding claim, wherein the controller (120) is configured to command the ultraviolet light source (110) to turn off prior to completion of at least one of the first dosage cycle or the second dosage cycle in response receiving a signal from the lavatory sensor indicating an occupant has entered the lavatory.

5. The disinfecting system of any preceding claim, wherein the controller (120) is configured to add one to the number of consecutive skipped cycles if the ultraviolet light source (110) is commanded by the controller (120) to turn off prior to completion of at least one of the first dosage cycle or the second dosage cycle, and/or wherein the controller (120) is configured to set the number of consecutive skipped cycles at zero if at least one of the first dosage cycle or the second dosage cycle is completed.

6. An article of manufacture including a tangible, non-transitory computer-readable storage medium having instructions stored thereon for controlling a disinfecting system for a lavatory, the instructions, in response to execution by a controller (120), cause the controller (120) to perform operations comprising:
identifying, by the controller (120), a disinfection initiation event;
comparing, by the controller (120), a consecutive skipped cycles count to a threshold number; and
commanding, by the controller (120), a ultraviolet light source (110) to implement at least one of a first dosage cycle or a second dosage cycle based on the comparison of the consecutive skipped cycles count to the threshold number, wherein the controller (120) is configured to implement the first dosage cycle if the consecutive skipped cycles count is less than the threshold number, and wherein the controller (120) is configured to implement the second dosage cycle if the consecutive skipped cycles count is greater than or equal to the threshold number.

7. The article of claim 6, wherein the operations further comprise starting, by the controller (120), a motion timer, in response to identifying the disinfection initiation event, and optionally wherein the operations further comprise adding, by the controller (120), one to the consecutive skipped cycles count if an interruption event is detected by the controller (120) prior to the motion timer exceeding a threshold time.

8. The article of claim 6 or 7, wherein the operations further comprise receiving, by the controller (120), a signal from a lavatory sensor configured to detect at least one of a toilet flush, a door latch position, motion in the lavatory, or a level of light in the lavatory.

9. The article of claim 8, wherein the controller (120) is configured to identify the disinfection initiation event based on the signal from the lavatory sensor, and/or wherein the operations further comprise:
identifying, by the controller (120), an interruption event based on the signal from the lavatory sensor;
commanding, by the controller (120), the ultraviolet light source (110) to stop the at least one of the first dosage cycle of the second dosage cycle in response to identifying the interruption event; and
adding, by the controller (120), one to the consecutive skipped cycles count.

10. The article of any of claims 6 to 9, wherein the operations further comprise setting, by the controller (120), the consecutive skipped cycles count to zero if at least one of the first dosage cycle or the second dosage cycle is completed.

11. The article of any of claims 6 to 10, wherein the operations further comprise commanding, by the controller (120), a display to output a message conveying an amount of time before the at least one of the first dosage cycle or the second dosage cycle is completed.

12. The article of any of claims 6 to 11, wherein the first dosage cycle is configured to achieve a 1 log reduction of a target pathogen, and wherein the second dosage cycle is configured to achieve a 3 log reduction of the target pathogen.

13. A method of disinfecting a lavatory, comprising:
identifying an ultraviolet disinfection initiation event;
starting a motion timer;
comparing a consecutive skipped cycles count to a threshold number if the motion timer exceeds a threshold time; and
implementing at least one of a first dosage cycle or a second dosage cycle based on the comparison of the consecutive skipped cycles count to the threshold number, wherein the first dosage cycle is implemented if the consecutive skipped cycles count is less than the threshold number, and wherein the second dosage cycle is implemented if the consecutive skipped cycles count is greater than or equal to the threshold number.

14. The method of claim 13, further comprising adding one to the consecutive skipped cycles count if an interruption event is detected prior to the motion timer exceeding the threshold time.

15. The method of claim 13 or 14, further comprising adding one to the consecutive skipped cycles count if an interruption event is detected prior to the at least one of the first dosage cycle or the second dosage cycle being completed, and optionally further comprising setting the consecutive skipped cycles count to zero if the at least one of the first dosage cycle or the second dosage cycle is completed.

## Patentansprüche

1. Desinfektionssystem für einen Waschraum, umfassend:
eine Ultraviolettlichtquelle (110);
eine Steuerung (120) in betriebsfähiger Verbindung mit der Ultraviolettlichtquelle (110); und
einen Waschraumsensor, der betriebsfähig mit der Steuerung (120) gekoppelt ist, wobei die Steuerung (120) dazu konfiguriert ist, eine Anzahl von aufeinanderfolgenden übersprungenen Zyklen zu verfolgen, und wobei die Steuerung (120) dazu konfiguriert ist, einen ersten Dosierzyklus umzusetzen, wenn die Anzahl von aufeinanderfolgenden übersprungenen Zyklen kleiner als eine Schwellenanzahl ist, und wobei die Steuerung (120) dazu konfiguriert ist, einen zweiten Dosierzyklus umzusetzen, wenn die Anzahl von aufeinanderfolgenden übersprungenen Zyklen größer oder gleich der Schwellenanzahl ist.

2. Desinfektionssystem nach Anspruch 1, wobei der Waschraumsensor mindestens eines von einem Spülsensor, einem Türklinkenpositionssensor, einem Bewegungssensor oder einem Lichtsensor umfasst.

3. Desinfektionssystem nach Anspruch 2, wobei der erste Dosierzyklus dazu konfiguriert ist, eine 1-log-Reduzierung eines Zielpathogens zu erreichen, und/oder wobei der zweite Dosierzyklus dazu konfiguriert ist, eine 3-log-Reduzierung des Zielpathogens zu erreichen.

4. Desinfektionssystem nach einem der vorhergehenden Ansprüche, wobei die Steuerung (120) dazu konfiguriert ist, die Ultraviolettlichtquelle (110) anzuweisen, sich vor Fertigstellung von mindestens einem von dem ersten Dosierzyklus oder dem zweiten Dosierzyklus als Reaktion auf ein Empfangen eines Signals von dem Waschraumsensor, das angibt, dass ein Benutzer den Waschraum betreten hat, abzuschalten.

5. Desinfektionssystem nach einem der vorhergehenden Ansprüche, wobei die Steuerung (120) dazu konfiguriert ist, der Anzahl von aufeinanderfolgenden übersprungenen Zyklen eins hinzuzufügen, wenn die Ultraviolettlichtquelle (110) durch die Steuerung (120) angewiesen wird, sich vor Fertigstellung von mindestens einem von dem ersten Dosierzyklus oder dem zweiten Dosierzyklus abzuschalten, und/oder wobei die Steuerung (120) dazu konfiguriert ist, die Anzahl von aufeinanderfolgenden übersprungenen Zyklen auf null zu setzen, wenn mindestens einer von dem ersten Dosierzyklus oder dem zweiten Dosierzyklus fertiggestellt ist.

6. Herstellungserzeugnis, das ein materielles, nicht flüchtiges, computerlesbares Speichermedium beinhaltet, das darauf gespeicherte Anweisungen zum Steuern eines Desinfektionssystems für einen Waschraum aufweist, wobei die Anweisungen als Reaktion auf die Ausführung durch eine Steuerung (120) die Steuerung (120) veranlassen, Vorgänge durchzuführen, die Folgendes umfassen:
Identifizieren eines Desinfektionsaufnahmeereignisses durch die Steuerung (120);
Vergleichen einer Zahl von aufeinanderfolgenden übersprungenen Zyklen mit einer Schwellenanzahl durch die Steuerung (120); und Anweisen einer Ultraviolettlichtquelle (110) durch die Steuerung (120), mindestens einen von einem ersten Dosierzyklus oder einem zweiten Dosierzyklus basierend auf dem Vergleich der Zahl von aufeinanderfolgenden übersprungenen Zyklen mit der Schwellenanzahl umzusetzen, wobei die Steuerung (120) dazu konfiguriert ist, den ersten Dosierzyklus umzusetzen, wenn die Zahl von aufeinanderfolgenden übersprungenen Zyklen kleiner als die Schwellenanzahl ist,
und wobei die Steuerung (120) dazu konfiguriert ist, den zweiten Dosierzyklus umzusetzen, wenn die Zahl von aufeinanderfolgenden übersprungenen Zyklen größer oder gleich der Schwellenanzahl ist.

7. Erzeugnis nach Anspruch 6, wobei die Vorgänge ferner Starten eines Bewegungszeitgebers durch die Steuerung (120) als Reaktion auf ein Identifizieren des Desinfektionsaufnahmeereignisses umfassen, und wobei die Vorgänge optional ferner Hinzufügen von eins durch die Steuerung (120) zu der Zahl von aufeinanderfolgenden übersprungenen Zyklen umfassen, wenn ein Unterbrechungsereignis durch die Steuerung (120) erkannt wird, bevor der Bewegungszeitgeber eine Schwellenzeit überschreitet.

8. Erzeugnis nach Anspruch 6 oder 7, wobei die Vorgänge ferner Empfangen eines Signals durch die Steuerung (120) von einem Waschraumsensor umfassen, der dazu konfiguriert ist, mindestens eines von einer Toilettenspülung, einer Türklinkenposition, einer Bewegung in dem Waschraum oder einer Lichtstärke in dem Waschraum zu erkennen.

9. Erzeugnis nach Anspruch 8, wobei die Steuerung (120) dazu konfiguriert ist, das Desinfektionsaufnahmeereignis basierend auf dem Signal von dem Waschraumsensor zu identifizieren, und/oder wobei die Vorgänge ferner Folgendes umfassen:
Identifizieren eines Unterbrechungsereignisses durch die Steuerung (120) basierend auf dem Signal von dem Waschraumsensor;
Anweisen der Ultraviolettlichtquelle (110) durch die Steuerung (120), den mindestens einen von dem ersten Dosierzyklus oder dem zweiten Dosierzyklus zu stoppen, als Reaktion auf das Identifizieren des Unterbrechungsereignisses; und
Hinzufügen von eins zu der Zahl von aufeinanderfolgenden übersprungenen Zyklen durch die Steuerung (120).

10. Erzeugnis nach einem der Ansprüche 6 bis 9, wobei die Vorgänge ferner Setzen der Zahl von aufeinanderfolgenden übersprungenen Zyklen auf null durch die Steuerung (120) umfassen, wenn mindestens einer von dem ersten Dosierzyklus oder dem zweite Dosierzyklus fertiggestellt ist.

11. Erzeugnis nach einem der Ansprüche 6 bis 10, wobei die Vorgänge ferner Anweisen einer Anzeige durch die Steuerung (120) umfassen, eine Meldung auszugeben, die eine Zeitspanne ausdrückt, bevor mindestens einer von dem ersten Dosierzyklus oder dem zweiten Dosierzyklus fertiggestellt ist.

12. Erzeugnis nach einem der Ansprüche 6 bis 11, wobei der erste Dosierzyklus dazu konfiguriert ist, eine 1-log-Reduzierung eines Zielpathogens zu erreichen, und wobei der zweite Dosierzyklus dazu konfiguriert ist, eine 3-log-Reduzierung des Zielpathogens zu erreichen.

13. Verfahren zum Desinfizieren eines Waschraums, umfassend:
Identifizieren eines Aufnahmeereignisses einer ultravioletten Desinfektion;
Starten eines Bewegungszeitgebers;
Vergleichen einer Zahl von aufeinanderfolgenden übersprungenen Zyklen mit einer Schwellenanzahl, wenn der Bewegungszeitgeber eine Schwellenzeit überschreitet; und
Umsetzen von mindestens einem von einem ersten Dosierzyklus oder einem zweiten Dosierzyklus basierend auf dem Vergleich der Zahl von aufeinanderfolgenden übersprungenen Zyklen mit der Schwellenanzahl, wobei der erste Dosierzyklus umgesetzt wird, wenn die Zahl von aufeinanderfolgenden übersprungenen Zyklen kleiner als die Schwellenanzahl ist, und wobei der zweite Dosierzyklus umgesetzt wird, wenn die Zahl von aufeinanderfolgenden übersprungenen Zyklen größer oder gleich der Schwellenanzahl ist.

14. Verfahren nach Anspruch 13, ferner Hinzufügen von eins zu der Zahl von aufeinanderfolgenden übersprungenen Zyklen umfassend, wenn ein Unterbrechungsereignis erkannt wird, bevor der Bewegungszeitgeber die Schwellenzeit überschreitet.

15. Verfahren nach Anspruch 13 oder 14, ferner Hinzufügen von eins zu der Zahl von aufeinanderfolgenden übersprungenen Zyklen umfassend, wenn ein Unterbrechungsereignis erkannt wird, bevor der mindestens eine von dem ersten Dosierzyklus oder dem zweiten Dosierzyklus fertiggestellt ist, und optional ferner Setzen der Zahl von aufeinanderfolgenden übersprungenen Zyklen auf null umfassend, wenn der mindestens eine von dem ersten Dosierzyklus oder dem zweiten Dosierzyklus fertiggestellt ist.

## Revendications

1. Système de désinfection pour toilettes, comprenant :
une source de lumière ultraviolette (110) ;
un dispositif de commande (120) en communication fonctionnelle avec la source de lumière ultraviolette (110) ; et
un capteur de toilettes couplé de manière fonctionnelle au dispositif de commande (120), dans lequel le dispositif de commande (120) est configuré pour suivre un nombre de cycles consécutifs sautés, et dans lequel le dispositif de commande (120) est configuré pour mettre en œuvre un premier cycle de dosage si le nombre de cycles consécutifs sautés est inférieur à un nombre seuil, et dans lequel le dispositif de commande (120) est configuré pour mettre en œuvre un second cycle de dosage si le nombre de cycles consécutifs sautés est supérieur ou égal au nombre seuil.

2. Système de désinfection selon la revendication 1, dans lequel le capteur de toilettes comprend au moins l'un parmi un capteur de chasse d'eau, un capteur de position de verrou de porte, un capteur de mouvement ou un capteur de lumière.

3. Système de désinfection selon la revendication 2, dans lequel le premier cycle de dosage est configuré pour atteindre une réduction de 1 log d'un pathogène cible, et/ou dans lequel le second cycle de dosage est configuré pour atteindre une réduction de 3 log du pathogène cible.

4. Système de désinfection selon une quelconque revendication précédente, dans lequel le dispositif de commande (120) est configuré pour commander à la source de lumière ultraviolette (110) de s'éteindre avant la fin d'au moins l'un parmi le premier cycle de dosage ou le second cycle de dosage en réponse à la réception d'un signal en provenance du capteur de toilettes indiquant qu'un occupant est entré dans les toilettes.

5. Système de désinfection selon une quelconque revendication précédente, dans lequel le dispositif de commande (120) est configuré pour ajouter un au nombre de cycles consécutifs sautés si le dispositif de commande (120) commande à la source de lumière ultraviolette (110) de s'éteindre avant la fin d'au moins l'un parmi le premier cycle de dosage ou le second cycle de dosage, et/ou dans lequel le dispositif de commande (120) est configuré pour définir le nombre de cycles consécutifs sautés à zéro si au moins l'un parmi le premier cycle de dosage ou le second cycle de dosage est terminé.

6. Article manufacturé comportant un support de stockage lisible par ordinateur, tangible et non transitoire sur lequel sont stockées des instructions destinées à commander un système de désinfection pour des toilettes, les instructions, en réponse à l'exécution par un dispositif de commande (120), amènent le dispositif de commande (120) à effectuer des opérations comprenant :
l'identification, par le dispositif de commande (120), d'un événement d'initiation de désinfection ;
la comparaison, par le dispositif de commande (120), d'un décompte de cycles consécutifs sautés avec un nombre seuil ; et la commande, par le dispositif de commande (120), d'une source de lumière ultraviolette (110) pour mettre en œuvre au moins l'un parmi un premier cycle de dosage ou un second cycle de dosage sur la base de la comparaison du décompte de cycles consécutifs
sautés avec le nombre seuil, dans lequel le dispositif de commande (120) est configuré pour mettre en œuvre le premier cycle de dosage si le décompte de cycles consécutifs sautés est inférieur au nombre seuil, et dans lequel le dispositif de commande (120) est configuré pour mettre en œuvre le second cycle de dosage si le décompte de cycles consécutifs sautés est supérieur ou égal au nombre seuil.

7. Article selon la revendication 6, dans lequel les opérations comprennent en outre le démarrage, par le dispositif de commande (120), d'un temporisateur de mouvement, en réponse à l'identification de l'événement d'initiation de désinfection, et éventuellement, dans lequel les opérations comprennent en outre l'ajout, par le dispositif de commande (120), d'un au décompte de cycles consécutifs sautés si un événement d'interruption est détecté par le dispositif de commande (120) avant que le temporisateur de mouvement ne dépasse un temps seuil.

8. Article selon la revendication 6 ou 7, dans lequel les opérations comprennent en outre la réception, par le dispositif de commande (120), d'un signal provenant d'un capteur de toilettes configuré pour détecter au moins l'un parmi une chasse d'eau, une position de verrouillage de porte, un mouvement dans les toilettes, ou un niveau de lumière dans les toilettes.

9. Article selon la revendication 8, dans lequel le dispositif de commande (120) est configuré pour identifier l'événement d'initiation de désinfection sur la base du signal provenant du capteur de toilettes, et/ou dans lequel les opérations comprennent en outre :
l'identification, par le dispositif de commande (120), d'un événement d'interruption sur la base du signal provenant du capteur de toilettes ;
le fait de commander, par le dispositif de commande (120), à la source de lumière ultraviolette (110) d'arrêter l'au moins un parmi le premier cycle de dosage du second cycle de dosage en réponse à l'identification de l'événement d'interruption ; et
l'ajout, par le dispositif de commande (120), d'un au décompte de cycles consécutifs sautés.

10. Article selon l'une quelconque des revendications 6 à 9, dans lequel les opérations comprennent en outre la définition, par le dispositif de commande (120), du décompte de cycles consécutifs sautés sur zéro si au moins l'un parmi le premier cycle de dosage ou le second cycle de dosage est terminé.

11. Article selon l'une quelconque des revendications 6 à 10, dans lequel les opérations comprennent en outre la commande, par le dispositif de commande (120), d'un affichage pour émettre un message transmettant une durée avant que l'au moins un parmi le premier cycle de dosage ou le second cycle de dosage est terminé.

12. Article selon l'une quelconque des revendication 6 à 11, dans lequel le premier cycle de dosage est configuré pour atteindre une réduction de 1 log d'un pathogène cible, et dans lequel le second cycle de dosage est configuré pour atteindre une réduction de 3 log du pathogène cible.

13. Procédé de désinfection de toilettes, comprenant :
l'identification d'un événement d'initiation de désinfection par ultraviolets ;
le démarrage d'un temporisateur de mouvement ;
la comparaison d'un décompte de cycles consécutifs sautés avec un nombre seuil si le temporisateur de mouvement dépasse un temps seuil ; et
la mise en œuvre d'au moins l'un parmi un premier cycle de dosage ou un second cycle de dosage sur la base de la comparaison du décompte de cycles consécutifs sautés avec le nombre seuil, dans lequel le premier cycle de dosage est mis en œuvre si le décompte de cycles consécutifs sautés est inférieur au nombre seuil, et dans lequel le second cycle de dosage est mis en œuvre si le décompte de cycles consécutifs sautés est supérieur ou égal au nombre seuil.

14. Procédé selon la revendication 13, comprenant en outre l'ajout d'un au décompte de cycles consécutifs sautés si un événement d'interruption est détecté avant que le temporisateur de mouvement ne dépasse le temps seuil.

15. Procédé selon la revendication 13 ou 14, comprenant en outre l'ajout d'un au décompte de cycles consécutifs sautés si un événement d'interruption est détecté avant que l'au moins un parmi le premier cycle de dosage ou le second cycle de dosage soit terminé, et comprenant éventuellement en outre la définition du décompte de cycles consécutifs sautés à zéro si l'au moins un parmi le premier cycle de dosage ou le second cycle de dosage est terminé.
